# NEW EUROPEAN PATENT SPECIFICATION

(11) **EP 2 734 175 B2**
(45) Date of publication and mention of the opposition decision: **04.09.2024**
(45) Mention of the grant of the patent: 12.12.2018
(21) Application number: 12733763.2
(22) Date of filing: 12.07.2012
(51) Int. Cl.: A61K 8/19, A61Q 15/00, A61K 8/20

(54) **USE, AS ANTIPERSPIRANT, OF A POLYVALENT CATION SALT WITHOUT ALUMINIUM HALIDE ANTIPERSPIRANT OR COMPOUND CAPABLE OF REACTING WITH SAID SALT IN ORDER TO PRODUCE AN ANTIPERSPIRANT EFFECT**
VERWENDUNG EINES MEHRWERTIGEN KATIONENSALZES OHNE EIN ALUMINIUMHALID-ANTIPERSPIRANT ALS ANTIPERSPIRANT ODER VERBINDUNG ZUR REAKTION MIT DIESEM SALZ ZWECKS HERSTELLUNG EINER SCHWEISSHEMMENDEN WIRKUNG
UTILISATION, COMME ANTI-TRANSPIRANT, D'UN SEL DE CATION MULTIVALENT SANS ANTI-TRANSPIRANT DE TYPE HALOGÉNURE D'ALUMINIUM OU COMPOSÉ APTE À RÉAGIR AVEC LEDIT SEL AFIN DE PRODUIRE UN EFFET ANTI-TRANSPIRANT

(30) Priority: 22.07.2011 FR 1156660; 26.07.2011 US 201161511708 P
(43) Date of publication of application: 28.05.2014
(73) Proprietor: L'OREAL, 75008 Paris (FR)
(72) Inventor: RAMOS-STANBURY, Laure, F-92330 Sceaux (FR); AUBRUN, Odile, F-92160 Antony (FR)
(74) Representative: L'Oreal
(86) International application number: PCT/EP2012/063719
(87) International publication number: WO 2013/013999

(56) References cited:
- EP-A1- 0 770 376
- WO-A1-00/10521
- WO-A1-97/15278
- WO-A1-99/52536
- WO-A2-01/78658
- CA-A- 1 076 030
- DE-T2- 9 600 120
- GB-A- 2 271 716
- US-A- 3 996 346
- US-A- 4 822 596
- US-A- 5 512 274
- US-A- 5 955 065
- US-A1- 2007 212 313
- US-B1- 6 605 288
- Brun, R; Manuila, L; Experiences on perspiration; Dermatologica, Volume 104/4-5 (1952) 267-72.
- "Secret" Anti Perspirant Deodorant, Mintel, database accession number 1182147, added to the database 09-2009
- Jungermann, E.; Antiperspirants: New Trends in Formulation and Testing Technology; J. Soc. Cosmet. Chem. 25 (11 -1974) 621-638.
- DATABASE gnpd [online] mintel; September 2009 (2009-09-01), "Anti Perspirant Deodorant", XP002679669, Database accession no. 1182147
- DATABASE gnpd [online] mintel; September 2009 (2009-09-01), "Clear Shield Antiperspirant Deodorant Stick", XP002679670, Database accession no. 1168989
- DATABASE gnpd [online] mintel; January 2010 (2010-01-01), "Everyday Deodorant", XP002679671, Database accession no. 1243385
- DATABASE gnpd [online] mintel; January 2004 (2004-01-01), "Rive Gauche pour Homme deodorant", XP002679672, Database accession no. 248582
- DATABASE gnpd [online] mintel; December 2009 (2009-12-01), "Deodorant Stick", XP002679673, Database accession no. 1223114
- DATABASE gnpd [online] mintel; November 2006 (2006-11-01), "Fragrance Free Aluminium Free Deodorant", XP002679674, Database accession no. 621053
- DATABASE gnpd [online] mintel; November 2008 (2008-11-01), "Deodorant", XP002679675, Database accession no. 1011539
- DATABASE gnpd [online] mintel; October 2007 (2007-10-01), "Green Forest Organic Deodorant", XP002679676, Database accession no. 789399

## Description

The present invention relates to a cosmetic method for treating human perspiration and optionally body odours resulting from perspiration, which comprises the single-step application to the skin of a composition comprising, in a cosmetically acceptable medium, at least one polyvalent metal cation salt which is calcium chloride; said composition containing no aluminium and/or zirconium antiperspirant halogenated salt nor compound capable of reacting with said polyvalent metal cation salt(s) in order to produce together on the skin, *in situ,* an antiperspirant effect.

The armpits and also certain other parts of the body are generally the site of much discomfort that may arise directly or indirectly from perspiration. This perspiration often leads to unpleasant and disagreeable sensations that are mainly due to the presence of sweat resulting from perspiration, which may, in certain cases, make the skin and clothing wet, especially in the region of the armpits or of the back, thus leaving visible marks. Moreover, the presence of sweat can give rise to the production of body odours, which are generally unpleasant. Finally, during its evaporation, sweat may also leave salts and/or proteins on the surface of the skin, which may result in whitish marks on the clothing. Such discomfort should not be disregarded, even in the case of moderate perspiration.

In the cosmetic field, it is thus well known to use, in topical application, antiperspirant products containing substances that have the effect of limiting or even preventing the flow of sweat in order to overcome the problems mentioned above. These products are generally available in the form of roll-ons, sticks, aerosols or sprays.

Antiperspirant substances generally consist of aluminium and/or zirconium chlorohydrates. These substances make it possible to reduce the flow of sweat by forming a plug in the sweat duct.

However, these substances can cause discomfort in some users in connection with the acidic pH of the formulations.

Furthermore, it has been observed that the antiperspirant effectiveness of these substances is limited when they are used alone. This means that these substances need to be applied to the skin on several occasions in order to obtain a satisfactory effective antiperspirant effect. Finally, these antiperspirant substances may also leave marks during their application, which has the consequence of staining the clothing.

To overcome all of the drawbacks mentioned above, it has been proposed to seek other effective active substances, which are well tolerated by the skin and easy to formulate, in order to replace all or some of the aluminium salts and/or aluminium and zirconium complexes.

The prior art describes antiperspirant compositions containing a calcium salt (CaCl₂).

In documents US 2007/0196303 (Reheis) and WO 2000/010512 (The Gillette Company), compositions are described that contain aluminium salts or aluminium/zirconium salts combined with a calcium salt in order to stabilize the aluminium species having the best *in vivo* efficacy.

Described in document US 5955065 (The Gillette Company) are antiperspirant compositions in aerosol form comprising aluminium salts and a calcium salt. The application does not describe the effectiveness of the compositions, and the activity of the calcium salt alone is not taught.

Described in document CA 2200498 are compositions containing calcium chloride, enabling odours to be eliminated.

The use of CaCl₂ acting by itself as an antiperspirant is not however described in the prior art.

Furthermore, documents are found in the literature that show that the salts of alkaline-earth metals such as calcium or magnesium are known for their lack of effectiveness in reducing perspiration. In the following document: RELLER and LUEDDERS (1977) Pharmacologic and Toxicologic Effects of Topically Applied Agents on the Eccrine Sweat Glands. In Advances in Modern Toxicology, Dermatotoxicology and Pharmacology (Ed. by F.N. Marzulli and H.I. Maibach), Vol. 4, p. 18. Hemisphere Publishing Co., Washington, the authors remark that only the metal salts capable of forming polymeric hydroxides at physiological pH or that have the ability to precipitate sweat proteins have an antiperspirant activity. Furthermore, it is clearly shown by means of an *in vitro* flow-plugging test (Table 6, page 23) and an *in vivo* efficacy test (Table 9, page 27 and Table 10, page 28) that calcium or magnesium salts are ineffective compared to aluminium salts.

In the document by Michniak, B. B., Studies on the Mechanism of Topical Anhidrosis due to Polyvalent Cations (International Journal of Cosmetic Science (1981), 3(1), 29-36), it is shown, on an animal model, that CaCl₂ is ineffective for reducing the sweat production induced by carbachol and even causes an increase of the flow of sweat.

Provision has already been made, in Patent FR 2 940 062, as alternative to aluminium and/or zirconium chlorohydrates, for a multicomponent agent for the treatment of human perspiration comprising two components which are intended to be mixed before application to the skin or to be applied to the skin simultaneously, separately or offset in time, said components being capable of reacting with one another to form one or more physical interactions in order to confer an antiperspirant effect. Mention is made, among the two components capable of reacting via physical bonding, of cations and anions and more particularly calcium chloride and ammonium hydrogen carbonate and also a mixture of zinc chloride and magnesium chloride with a terpolymer based on vinyl acetate, *tert*-butyl vinylbenzoate and crotonic acid.

Provision has already been made, in Application WO 2010/070143, as alternative to aluminium and/or zirconium chlorohydrates, for a multicomponent agent for the treatment of human perspiration comprising two components which are intended to be mixed before application to the skin or to be applied to the skin simultaneously, separately or offset in time, one of the components being capable of treating the skin and the other component being capable of acting on the skin once it has been treated, said combination making it possible to confer an antiperspirant effect.

Provision has already been made, in Application WO 2010/070139, as alternative to aluminium and/or zirconium chlorohydrates, for a multicomponent agent for the treatment of human perspiration comprising two components which are intended to be mixed before application to the skin or to be applied to the skin simultaneously, separately or offset in time, said components being capable of reacting with one another to form covalent bonds.

The antiperspirant effectiveness of these three methods is not completely satisfactory. In addition, the systems proposed in these patent applications require the use of a two-fold action or of an extemporaneous preparation, which remain restrictive for the consumers.

Thus, there therefore exists a real need to employ, on the skin, an agent intended for the treatment of human perspiration which substitutes for the conventional aluminium and/or zirconium halogenated salts, which does not exhibit the combination of disadvantages described above, that is to say which confers a satisfactory antiperspirant effect, in particular in terms of effectiveness and resistance to sweat, and which is well tolerated by the skin.

The Applicant has surprisingly discovered that this objective could be achieved by turning to a cosmetic method for treating human perspiration which comprises the single-step application to the skin of a composition comprising, in a cosmetically acceptable medium, at least one polyvalent metal cation salt, said composition containing no aluminium and/or zirconium antiperspirant halogenated salt nor compound capable of reacting with said polyvalent metal cation salt(s) in order to produce on the skin, *in situ,* an antiperspirant effect.

The present invention relates to a cosmetic method for treating human perspiration and optionally body odours resulting from perspiration, which comprises the single-step application to the skin of a non-extemporaneous composition comprising, in a cosmetically acceptable medium, at least one polyvalent metal cation salt which is calcium chloride ; said composition containing no aluminium and/or zirconium antiperspirant halogenated salt nor compound capable of reacting with said polyvalentmetal cation salt(s) in order to produce together on the skin, *in situ,* an antiperspirant effect. The present invention specifically relates to a non-therapeutic use of a cosmetic composition comprising, in a cosmetically acceptable medium, calcium chloride as antiperspirant agent, for treating human perspiration, in order to reduce the flow of sweat, said composition containing no aluminium and/or zirconium antiperspirant halogenated salt, nor compound capable of reacting with said calcium chloride in order to produce together on the skin, in situ, an antiperspirant effect.

The term "antiperspirant agent" means any substance or any composition which has the effect of reducing the flow of sweat and/or of reducing the sensation of dampness associated with human sweat, and/or of masking human sweat.

The term "cosmetically acceptable medium" means a medium that is compatible with the skin and/or its superficial body growths or mucous membranes, having a pleasant colour, odour and feel and not causing any unacceptable discomfort (stinging, tightness or redness) liable to discourage the consumer from using this composition.

The term "polyvalent metal cation" is understood to mean any ion chosen from alkaline-earth metals or transition metals comprising at least two positive electric charges and having a valency of at least 2 and preferably 2 or 3.

The expression "composition containing no aluminium and/or zirconium antiperspirant halogenated salt" is understood to mean any composition containing less than 1% or even less than 0.5% by weight relative to the total weight of the composition of aluminium and/or zirconium antiperspirant salt comprising in its structure at least one halogen atom (for example aluminium and/or zirconium chlorohydrate) and in particular that is free of said salt.

The expression "compound capable of reacting with said polyvalent metal cation salt(s) in order to produce together on the skin, *in situ,* an antiperspirant effect" is understood to mean any reactive compound such as:
(i) those described in Patent FR 2 940 062 capable of reacting with the metal cation salt(s) by means of one or more physical interactions, that is to say, by forming with one another one or more non-covalent bonds chosen from ionic bonds, hydrogen bonds, van der Waals bonds or hydrophobic bonds and more particularly those capable of reacting with the metal cation salt(s) by means of a cation/anion ionic reaction and giving rise to a precipitation or a flocculation. For example, in the case where the polyvalent cation salt is calcium chloride, the reactive compound may be a hydrogen phosphate salt such as ammonium hydrogen phosphate.
(ii) those described in Application WO 2010/070143 capable of cooperating with the polyvalent cation salt(s) in contact with the skin and of producing, *in situ,* an antiperspirant effect. The polyvalent cation salt(s) in accordance with the invention or else the reactive compound is capable of chemically or physically modifying the skin so that the other co-reactant can be active or so that its activity is strengthened on the pretreated skin in order to confer an antiperspirant effect;
(iii) those described in Application WO 2010/070139 and comprising one or more functions capable of reacting with the polyvalent cation salt(s) by means of covalent bonds so as to confer an antiperspirant effect.

The expression "composition containing no compound capable of reacting with said polyvalent metal cation salt(s) in order to produce together on the skin, *in situ,* an antiperspirant effect" is understood to mean any composition containing less than 1% or even less than 0.5% by weight relative to the total weight of the composition, of said anion salt, and in particular that is free of said anion salt, for example ammonium hydrogen carbonate when the polyvalent cation salt is the calcium chloride.

### POLYVALENT CATION

The polyvalent cation used in accordance with the invention is calcium chloride.

The concentration of calcium chloride in the composition preferably varies from 0.1% to 20% and more preferentially from 0.5% to 15% by weight relative to the total weight of the composition.

### GALENIC FORMS

The compositions according to the invention may be in any galenic form conventionally used for topical application and especially in the form of aqueous gels, or aqueous or aqueous-alcoholic solutions. By adding a fatty or oily phase, they may also be provided in the form of dispersions of lotion type, of emulsions of liquid or semi-liquid consistency of milk type, obtained by dispersing a fatty phase in an aqueous phase (O/W) or conversely (W/O), or of suspensions or emulsions of soft, semi-solid or solid consistency of the cream or gel type, or alternatively of multiple emulsions (W/O/W or O/W/O), of microemulsions, of vesicular dispersions of ionic and/or nonionic type, or of wax/aqueous phase dispersions. These compositions are prepared according to the usual methods.

The compositions may especially be packaged in pressurized form in an aerosol device or in a pump dispenser; packaged in a device equipped with an openwork wall, especially a grille; packaged in a device equipped with a ball applicator ("roll-on"); packaged in the form of sticks or in the form of loose or compacted powder. In this regard, they contain the ingredients generally used in products of this type, which are well known to those skilled in the art.

According to another particular form of the invention, the compositions according to the invention may be anhydrous.

The term "anhydrous composition" means a composition containing less than 2% by weight of water, indeed even less than 0.5% of water, and especially devoid of water, the water not being added during the preparation of the composition but corresponding to the residual water contributed by the mixed ingredients. According to another particular form of the invention, the compositions A and/or B according to the invention may be solid, in particular in stick form, or in the form of loose or compacted powder.

The term "solid composition" means that the measurement of the maximum force measured by texturometry during the penetration of a probe into the sample of formula must be at least equal to 0.25 newtons, in particular at least equal to 0.30 newtons and especially at least equal to 0.35 newtons, assessed under precise measurement conditions as follows.

The formulae are poured hot into jars 4 cm in diameter and 3 cm deep. Cooling is performed at ambient temperature. The hardness of the formulae produced is measured after an interval of 24 hours. The jars containing the samples are characterized in texturometry using a texture analyser such as the machine sold by the company Rheo TA-XT2, according to the following protocol: a stainless-steel ball probe 5 mm in diameter is brought into contact with the sample at a speed of 1 mm/s. The measurement system detects the interface with the sample, with a detection threshold equal to 0.005 newtons. The probe penetrates 0.3 mm into the sample, at a speed of 0.1 mm/s. The measuring machine records the change in force measured in compression over time, during the penetration phase. The hardness of the sample corresponds to the average of the maximum force values detected during penetration, over at least three measurements.

### AQUEOUS PHASE

The compositions according to the invention intended for cosmetic use may comprise at least one aqueous phase. They are especially formulated as aqueous lotions or as water-in-oil or oil-in-water emulsions or as multiple emulsions (oil-in-water-in-oil or water-in-oil-in-water triple emulsions (such emulsions are known and described, for example, by C. Fox in "Cosmetics and Toiletries" - November 1986 - Vol. 101 - pages 101-112)).

The aqueous phase(s) of said compositions contain water and generally other water-soluble or water-miscible solvents. The water-soluble or water-miscible solvents comprise short chain, for example C₁-C₄, monoalcohols, such as ethanol or isopropanol; diols or polyols, for instance ethylene glycol, 1,2-propylene glycol, 1,3-butylene glycol, hexylene glycol, diethylene glycol, dipropylene glycol, 2-ethoxyethanol, diethylene glycol monomethyl ether, triethylene glycol monomethyl ether and sorbitol. Propylene glycol and glycerol, propane-1,3-diol, will be used more particularly.

The aqueous phase(s) of said compositions may contain one or more hydrophilic thickeners or gelling agents.

The hydrophilic thickeners or gelling agents may be chosen from carboxyvinyl polymers such as the Carbopol products (carbomers) and the Pemulen products (acrylate/C₁₀-C₃₀-alkyl acrylate copolymer); polyacrylamides, for instance the crosslinked copolymers sold under the names Sepigel 305 (CTFA name: polyacrylamide/C₁₃₋₁₄ isoparaffin/Laureth 7) or Simulgel 600 (CTFA name: acrylamide/sodium acryloyldimethyltaurate copolymer/isohexadecane/polysorbate 80) by the company SEPPIC; 2-acrylamido-2-methylpropanesulfonic acid polymers and copolymers, optionally crosslinked and/or neutralized, for instance poly(2-acrylamido-2-methylpropanesulfonic acid) sold by the company Hoechst under the trade name Hostacerin AMPS (CTFA name: ammonium polyacryloyldimethyltaurate or Simulgel 800 sold by the company SEPPIC (CTFA name: sodium polyacryloyldimethyltaurate/polysorbate 80/sorbitan oleate); copolymers of 2-acrylamido-2-methylpropanesulfonic acid and of hydroxyethyl acrylate, for instance Simulgel NS and Sepinov EMT 10 sold by the company SEPPIC; cellulose derivatives such as hydroxyethyl cellulose or cetyl hydroxyethyl cellulose; polysaccharides and especially gums such as xanthan gum and hydroxypropyl guar gums; silicas, for instance Bentone Gel MIO sold by the company NL Industries or Veegum Ultra sold by the company Polyplastic.

The hydrophilic thickeners or gelling agents may also be cationic, for instance Polyquaternium-37 sold under the name Salcare SC95 (Polyquaternium-37 (and) Mineral Oil (and) PPG-1 Trideceth-6) or Salcare SC96 (Polyquaternium-37 (and) Propylene Glycol Dicaprylate/Dicaprate (and) PPG-1 Trideceth-6) or other crosslinked cationic polymers, for instance those of the CTFA name Ethyl Acrylate/Dimethylaminoethyl Methacrylate Cationic Copolymer in emulsion.

### EMULSIFIERS

### a) Oil-in-water emulsifiers

Mention may be made, as emulsifiers which can be used in oil-in-water emulsions or oil-in-water-in-oil triple emulsions, of emulsifiers having an HLB of greater than or equal to 8, in particular ranging from 8 to 25.

Mention may be made, as examples, of the following emulsifiers:
non-ionic emulsifiers, such as oxyalkylenated (more particularly polyoxyethylenated) fatty acid esters of glycerol; oxyalkylenated fatty acid esters of sorbitan; oxyalkylenated (oxyethylenated and/or oxypropylenated) fatty acid esters; oxyalkylenated (oxyethylenated and/or oxypropylenated) fatty alcohol ethers; sugar esters such as sucrose stearate; and mixtures thereof, such as the mixture of glyceryl stearate and PEG-40 stearate.

Mention may also be made of fatty alcohol/alkylpolyglycoside emulsifying mixtures as described in Patent Applications WO 92/06778, WO 95/13863 and WO 98/47610, for instance the commercial products sold by the company SEPPIC under the name Montanov ^{®}.

### b) Water-in-oil emulsifiers

Mention may be made, among the emulsifiers which can be used in water-in-oil emulsions or water-in-oil-in-water triple emulsions, of emulsifiers having an HLB (hydrophilic/lipophilic balance) of less than or equal to 6, in particular ranging from 4 to 6.

Mention may be made, as examples of first emulsifiers, of polyol fatty esters, in particular glycerol or sorbitol fatty esters, and in particular polyol isostearic, oleic and ricinoleic esters, such as the mixture of petrolatum, polyglyceryl- 3 oleate, glyceryl isostearate, hydrogenated castor oil and ozokerite sold under the name Protegin W^{®} by the company Goldschmidt, sorbitan isostearate, polyglyceryl diisostearate, polyglyceryl-2 sesquiisostearate; saccharide esters and ethers, such as "Methyl glucose dioleate"; fatty esters, such as magnesium lanolate; dimethicone copolyols and alkyl dimethicone copolyols.

Mention may be made, by way of example, of the alkyl dimethicone copolyols corresponding to the following formula (I): 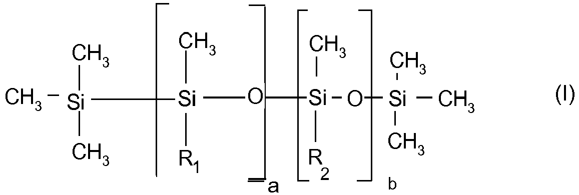 in which:
R₁ denotes a linear or branched C₁₂-C₂₀ and preferably C₁₂-C₁₈ alkyl group;
R₂ denotes the group: --CₙH₂ₙ--(-OC₂H₄-)ₓ--(-OC₃H₆-)_{y}--O-R₃;
R₃ denotes a hydrogen atom or a linear or branched alkyl radical comprising from 1 to 12 carbon atoms;
a is an integer ranging from 1 to about 500;
b denotes an integer ranging from 1 to about 500;
n is an integer ranging from 2 to 12 and preferably from 2 to 5;
x denotes an integer ranging from 1 to about 50 and preferably from 1 to 30;
y denotes an integer ranging from 0 to about 49 and preferably from 0 to 29, with the proviso that, when y is other than zero, the ratio x/y is greater than 1 and preferably varies from 2 to 11.

Among the alkyl dimethicone copolyol emulsifiers of formula (I) that are preferred, mention will be made more particularly of Cetyl PEG/PPG-10/1 Dimethicone and more particularly the mixture Cetyl PEG/PPG-10/1 Dimethicone and Dimethicone (INCI name), for instance the product sold under the trade name Abil EM90 by the company Goldschmidt, or alternatively the mixture (Polyglyceryl-4 Stearate and Cetyl PEG/PPG-10 (and) Dimethicone (and) Hexyl Laurate), for instance the product sold under the trade name Abil WE09 by the same company.
Among the water-in-oil emulsifiers, mention may also be made of the dimethicone copolyols corresponding to formula (II) below: 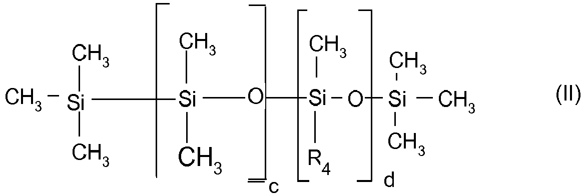 in which:
R₄ denotes the group:--CₘH₂ₘ--(-OC₂H₄-)ₛ--(-OC₃H₆-)ₜ--O-R₅,
R₅ denotes a hydrogen atom or a linear or branched alkyl radical comprising from 1 to 12 carbon atoms;
c is an integer ranging from 1 to about 500;
d denotes an integer ranging from 1 to about 500;
m is an integer ranging from 2 to 12 and preferably from 2 to 5;
s denotes an integer ranging from 1 to about 50 and preferably from 1 to 30;
t denotes an integer ranging from 0 to about 50 and preferably from 0 to 30; with the proviso that the sum s+t is greater than or equal to 1.

Among these preferential dimethicone copolyol emulsifiers of formula (II), use will particularly be made of PEG-18/PPG-18 Dimethicone and more particularly the mixture Cyclopentasiloxane (and) PEG-18/PPG-18 Dimethicone (INCI name), such as the product sold by the company Dow Corning under the trade name Silicone DC 5225 C or KF-6040 from the company Shin Etsu.

According to a particularly preferred form, use will be made of a mixture of at least one emulsifier of formula (I) and of at least one emulsifier of formula (II).

Use will be made more particularly of a mixture of PEG-18/PPG-18 Dimethicone and Cetyl PEG/PPG-10/1 Dimethicone and even more particularly of a mixture of (Cyclopentasiloxane (and) PEG-18/PPG-18 Dimethicone) and of Cetyl PEG/PPG-10/1 Dimethicone and Dimethicone or of (Polyglyceryl-4 Stearate and Cetyl PEG/PPG-10 (and) Dimethicone (and) Hexyl Laurate).

Among the water-in-oil emulsifiers, mention may also be made of non-ionic emulsifiers derived from fatty acids and polyols, alkylpolyglycosides (APGs), sugar esters and mixtures thereof.

As non-ionic emulsifiers derived from fatty acids and polyols, use may be made especially of fatty acid esters of polyols, the fatty acid especially containing a C₈-C₂₄ alkyl chain, and the polyols being, for example, glycerol and sorbitan.

Fatty acid esters of polyols that may especially be mentioned include isostearic acid esters of polyols, stearic acid esters of polyols, and mixtures thereof, in particular isostearic acid esters of glycerol and/or sorbitan.

Stearic acid esters of polyols that may especially be mentioned include the polyethylene glycol esters, for instance PEG-30 Dipolyhydroxystearate, such as the product sold under the name Arlacel P135 by the company ICI.

Glycerol and/or sorbitan esters that may be mentioned, for example, include polyglyceryl isostearate, such as the product sold under the name Isolan GI 34 by the company Goldschmidt; sorbitan isostearate, such as the product sold under the name Arlacel 987 by the company ICI; sorbitan glyceryl isostearate, such as the product sold under the name Arlacel 986 by the company ICI, the mixture of sorbitan isostearate and polyglyceryl isostearate (3 mmol) sold under the name Arlacel 1690 by the company Uniqema, and mixtures thereof.

The emulsifier may also be chosen from alkylpolyglycosides with an HLB of less than 7, for example those represented by the general formula (1) below:

R-O-(G)ₓ (1)

in which R represents a branched and/or unsaturated alkyl radical comprising from 14 to 24 carbon atoms, G represents a reduced sugar comprising 5 or 6 carbon atoms, and x is a value ranging from 1 to 10 and preferably from 1 to 4, and G especially denotes glucose, fructose or galactose.

The unsaturated alkyl radical may comprise one or more ethylenically unsaturated groups, and in particular one or two ethylenically unsaturated groups.

As alkylpolyglycosides of this type, mention may be made of alkylpolyglucosides (G = glucose in formula (I)), and especially the compounds of formula (I) in which R more particularly represents an oleyl radical (unsaturated C₁₈ radical) or an isostearyl radical (saturated C₁₈ radical), G denotes glucose, x is a value ranging from 1 to 2, especially isostearyl glucoside or oleyl glucoside, and mixtures thereof. This alkylpolyglucoside may be used as a mixture with a coemulsifier, more especially with a fatty alcohol and especially a fatty alcohol containing the same fatty chain as that of the alkylpolyglucoside, i.e. comprising from 14 to 24 carbon atoms and containing a branched and/or unsaturated chain, and for example isostearyl alcohol when the alkylpolyglucoside is isostearyl glucoside, and oleyl alcohol when the alkylpolyglucoside is oleyl glucoside, optionally in the form of a self-emulsifying composition, as described, for example, in the document WO-A-92/06778. Use may be made, for example, of the mixture of isostearyl glucoside and isostearyl alcohol, sold under the name Montanov WO 18 by the company SEPPIC, and also the mixture of octyldodecanol and octyldodecyl xyloside sold under the name Fludanov 20X by the company SEPPIC.

Mention may also be made of succinic-terminated polyolefins, for instance esterified succinic-terminated polyisobutylenes and salts thereof, especially the diethanolamine salts, such as the products sold under the names Lubrizol 2724, Lubrizol 2722 and Lubrizol 5603 by the company Lubrizol or the commercial product Chemcinnate 2000.

Mention may also be made, among water-in-oil emulsifiers, of oxyalkylenated silicone elastomers, in particular polyoxyethylenated and/or polyoxypropylenated silicone elastomers and more particularly polyoxyethylenated silicone elastomers, such as those described in the documents US-A-5 236 986, US-A-5 412 004, US-A-5 837 793 and US-A-5 811 487. The polyoxyalkylenated silicone elastomer is preferably conveyed in the form of a gel in at least one hydrocarbon oil and/or one silicone oil. In these gels, the polyoxyalkylenated elastomer is often in the form of non-spherical particles.

As polyoxyethylenated silicone elastomers, it is possible to use those sold by the company Shin-Etsu under the names:
- KSG-21 (containing 27% active material. INCI name: Dimethicone/PEG-10 Dimethicone vinyl dimethicone crosspolymer),
- KSG-20 (containing 95% active material. INCI name: PEG-10 dimethicone crosspolymer),
- KSG-30 (containing 100% active material. INCI name: Lauryl PEG-15 Dimethicone vinyl dimethicone crosspolymer),
- KSG-31 (containing 25% active material. INCI name: Lauryl PEG-15 Dimethicone vinyl dimethicone crosspolymer),
- KSG-32 or KSG-42 or KSG-320 or KSG-30 (containing 25% active material. INCI name: Lauryl PEG-15 Dimethicone vinyl dimethicone crosspolymer),
- KSG-33 (containing 20% active material),
- KSG-210 (containing 25% active material. INCI name: Dimethicone/PEG-10/15 crosspolymer),
- KSG-310: lauryl-modified crosslinked polyoxyethylenated polydimethylsiloxane in mineral oil,
- KSG-330,
- KSG-340,
- X-226146 (containing 32% active material. INCI name: Dimethicone/PEG-10 Dimethicone vinyl dimethicone crosspolymer),
or those sold by the company Dow Corning under the names:
- DC9010 (containing 9% active material. INCI name: PEG-12 dimethicone crosspolymer)
- DC9011 (containing 11% active material).

These products are generally provided in the form of oily gels containing silicone elastomer particles.

KSG-210 (INCI name: Dimethicone/PEG-10/15 crosspolymer), which contains 25% silicone elastomer active material in silicone oil, is preferably used.

Mention may also be made, among water-in-oil emulsifiers, of polyglycerolated silicone elastomers. Such elastomers are described in particular in the document WO-A-2004/024798.

Use may be made, as polyglycerolated silicone elastomers, of those sold under the names:
- KSG-710 (containing 25% active material. INCI name: Dimethicone/Polyglycerin-3 Crosspolymer),
- KSG-810,
- KSG-820,
- KSG-830,
- KSG-840,
by the company Shin-Etsu.

Mention may be made, among the emulsifiers which can be used in oil-in-water emulsions or oil-in-water-in-oil triple emulsions, of emulsifiers having an HLB (hydrophilic/lipophilic balance) of greater than or equal to 8, in particular ranging from 8 to 25.

Mention may for example be made, as examples of O/W emulsifiers, of the following emulsifiers:
* as amphoteric emulsifiers, N-acylamino acids, such as N-alkylamino acetates and disodium cocoamphodiacetate, and amine oxides such as stearamine oxide;
* as anionic emulsifiers, acyl glutamates such as "disodium hydrogenated tallow glutamate" (Amisoft HS-21^{®} sold by the company AJINOMOTO); carboxylic acids and salts thereof such as sodium stearate; phosphoric esters and salts thereof such as "DEA oleth-10 phosphate"; sulfosuccinates such as "Disodium PEG-5 citrate lauryl sulfosuccinate" and "Disodium ricinoleamido MEA sulfosuccinate";
* as cationic emulsifiers, alkyl imidazolidiniums such as isostearyl ethylimidonium ethosulfate; ammonium salts such as N,N,N-trimethyl-1-docosanaminium chloride (behentrimonium chloride);
* as non-ionic emulsifiers, fatty alcohols, fatty acids, fatty amides, saccharide esters and ethers such as sucrose stearate, sucrose cocoate, and the mixture of sorbitan stearate and sucrose cocoate sold by the company ICI under the name Arlatone 2121^{®}; polyol esters, in particular glycerol or sorbitol esters, such as glyceryl stearate, polyglyceryl-6 stearate, sorbitan stearate; glycerol ethers; oxyethylenated and/or oxypropylenated ethers, such as the oxyethylenated, oxypropylenated ether of lauryl alcohol containing 25 oxyethylenated groups and 25 oxypropylenated groups (CTFA name "PPG-25 laureth-25") and the oxyethylenated ether of the mixture of C₁₂-C₁₅ fatty alcohols comprising 7 oxyethylenated groups (CTFA name "C₁₂-C₁₅ Pareth-7"); polymers of ethylene glycol, such as PEG-100; block copolymers of ethylene oxide and propylene oxide, such as SYNPERONIC PE F127 sold by ICI; and mixtures thereof.

The O/W emulsifier may also be chosen from emulsifying polymers such as the polymers and copolymers of vinyl alcohol, vinyl pyrrolidone, AMPS, acrylic, etc.

The total amount of emulsifiers in the composition will preferably be, in the composition according to the invention, at active material contents ranging from 1% to 8% by weight and more particularly from 2% to 6% by weight, relative to the total weight of the composition.

According to one particular form of the invention, the compositions A and/or B of the invention in the emulsion form can be prepared according to the phase inversion manufacturing technique. This technique is, in its principle, well known and is described in particular in the article "Phase Inversion Emulsification" by Th. Forster et al. which appeared in Cosmetics & Toiletries, vol. 106, December 1991, pages 49-52. Its principle is as follows: (i) A fatty phase, on the one hand, and an aqueous phase, on the other hand, are mixed with stirring in the presence of a suitable emulsifying system, said mixing being carried out at a temperature above the phase inversion temperature (PIT) of the medium, so as to obtain an emulsion of water-in-oil type. (ii) The temperature of the emulsion thus obtained is brought back to a temperature below said phase inversion temperature, whereby an ultrafine emulsion of oil-in-water type is obtained. (iii) Inorganic nanopigments are introduced during the implementation of stage (i) and/or on conclusion of stage (ii).

The systems suitable are emulsifiers of non-ionic type chosen from polyoxyethylenated and/or polyoxypropylenated fatty alcohols (i.e., compounds obtained by reaction between an aliphatic fatty alcohol, such as behenyl alcohol or cetyl alcohol, and ethylene oxide or propylene oxide or an ethylene oxide/propylene oxide mixture) and esters of fatty acids and of polyols, which are optionally polyoxyethylenated and/or polyoxypropylenated (i.e., compounds obtained by reaction of a fatty acid, such as stearic acid or oleic acid, with a polyol, such as, for example, an alkylene glycol or glycerol or a polyglycerol, optionally in the presence of ethylene oxide or propylene oxide or an ethylene oxide/propylene oxide mixture), or their mixtures. Furthermore and preferably, the emulsifying system selected will exhibit an overall HLB (HLB = Hydrophilic/Lipophilic Balance, within the meaning of Griffin; see J. Soc. Cosm. Chem., 1954 (vol. 5), pp 249-256; the equilibrium between the hydrophilic nature and the lipophilic nature of the surface-active agent) ranging from 9.5 to 11.5 approximately, advantageously about 10, so as to make it possible to obtain a phase inversion at a temperature below 90°C (PIT < 90°C). The content of emulsifying agent(s) is between 0.5% and 40% by weight and preferably between 2% and 10% by weight, relative to the total weight of the emulsion.

### FATTY PHASE

The compositions according to the invention may contain at least one water-immiscible organic liquid phase, known as a fatty phase. This phase generally comprises one or more hydrophobic compounds that render said phase water-immiscible. Said phase is liquid (in the absence of structuring agent) at ambient temperature (20-25°C). Preferentially, the water-immiscible organic liquid phase in accordance with the invention generally comprises at least one volatile oil and/or one non-volatile oil and optionally at least one structuring agent.

The term "oil" means a fatty substance that is liquid at ambient temperature (20-25°C) and atmospheric pressure (760 mmHg, i.e. 10⁵ Pa). The oil may be volatile or non-volatile.

Within the meaning of the invention, the term "volatile oil" means an oil that is capable of evaporating on contact with the skin or the keratin fibre in less than one hour, at ambient temperature and atmospheric pressure. The volatile oils of the invention are volatile cosmetic oils, which are liquid at ambient temperature, having a non-zero vapour pressure, at ambient temperature and atmospheric pressure, ranging in particular from 0.13 Pa to 40 000 Pa (10⁻³ to 300 mmHg), in particular ranging from 1.3 Pa to 13 000 Pa (0.01 to 100 mmHg) and more particularly ranging from 1.3 Pa to 1300 Pa (0.01 to 10 mmHg).

The term "non-volatile oil" means an oil that remains on the skin or the keratin fibre at ambient temperature and atmospheric pressure for at least several hours, and that especially has a vapour pressure of less than 10⁻³ mmHg (0.13 Pa).

The oil may be chosen from any physiologically acceptable oil and in particular cosmetically acceptable oil, especially mineral, animal, vegetable or synthetic oils; in particular volatile or non-volatile hydrocarbon oils and/or silicone oils and/or fluoro oils, and mixtures thereof.

More precisely, the term "hydrocarbon oil" means an oil mainly comprising carbon and hydrogen atoms and optionally one or more functional groups chosen from hydroxyl, ester, ether and carboxylic functional groups. Generally, the oil has a viscosity of from 0.5 to 100 000 mPa.s, preferably from 50 to 50 000 mPa.s and more preferably from 100 to 300 000 mPa.s.

As examples of volatile oils that may be used in the invention, mention may be made of:
- volatile hydrocarbon oils chosen from hydrocarbon oils containing from 8 to 16 carbon atoms, and especially C₈-C₁₆ isoalkanes of petroleum origin (also known as isoparaffins), for instance isododecane (also known as 2,2,4,4,6-pentamethylheptane), isodecane and isohexadecane, and for example the oils sold under the trade names Isopar or Permethyl, branched C₈-C₁₆ esters, isohexyl neopentanoate, and mixtures thereof. Other volatile hydrocarbon oils, for instance petroleum distillates, especially those sold under the name Shell Solt by the company Shell, may also be used; volatile linear alkanes, such as those described in Patent Application DE10 2008 012 457 from the company Cognis;
- volatile silicones, for instance volatile linear or cyclic silicone oils, especially those with a viscosity ≤ 8 centistokes (8310⁻⁶ m²/s) and especially containing from 2 to 7 silicon atoms, these silicones optionally comprising alkyl or alkoxy groups containing from 1 to 10 carbon atoms. As volatile silicone oils that may be used in the invention, mention may be made especially of octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane or dodecamethylpentasiloxane;
- and mixtures thereof.

Mention may also be made of volatile linear alkyltrisiloxane oils of general formula (I): 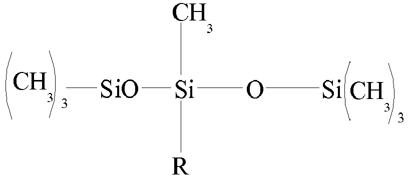 in which R represents an alkyl group containing from 2 to 4 carbon atoms, one or more hydrogen atoms of which may be replaced by a fluorine or chlorine atom.

Among the oils of general formula (I) that may be mentioned are:
3-butyl-1,1,1,3,5,5,5-heptamethyltrisiloxane,
3-propyl-1,1,1,3,5,5,5-heptamethyltrisiloxane, and
3-ethyl-1,1,1,3,5,5,5-heptamethyltrisiloxane,
corresponding to the oils of formula (I) for which R is, respectively, a butyl group, a propyl group or an ethyl group.

As examples of non-volatile oils that may be used in the invention, mention may be made of:
- hydrocarbon oils of animal origin, such as perhydrosqualene;
- hydrocarbon vegetable oils, such as liquid triglycerides of fatty acids having 4 to 24 carbon atoms, for instance heptanoic or octanoic acid triglycerides, or wheatgerm oil, olive oil, sweet almond oil, palm oil, rapeseed oil, cottonseed oil, alfalfa oil, poppy oil, pumpkin oil, marrow oil, blackcurrant oil, evening primrose oil, millet oil, barley oil, quinoa oil, rye oil, safflower oil, candlenut oil, passion flower oil, musk rose oil, sunflower oil, maize oil, soybean oil, grapeseed oil, sesame seed oil, hazelnut oil, apricot oil, macadamia oil, castor oil, avocado oil, caprylic/capric acid triglycerides, for instance those sold by the company Stéarineries Dubois or those sold under the names Miglyol 810, 812 and 818 by the company Sasol, jojoba oil or shea butter oil;
- linear or branched hydrocarbons, of mineral or synthetic origin, such as liquid paraffins and derivatives thereof, petroleum jelly, polydecenes, polybutenes, hydrogenated polyisobutene such as Parleam, or squalane;
- synthetic ethers containing from 10 to 40 carbon atoms;
- synthetic esters, especially of fatty acids, for instance the oils of formula R₁COOR₂ in which R₁ represents a linear or branched higher fatty acid residue containing from 1 to 40 carbon atoms and R₂ represents a hydrocarbon chain, which is especially branched, containing from 1 to 40 carbon atoms, with R₁ + R₂ ≥ 10, for instance purcellin oil (cetostearyl octanoate), isononyl isononanoate, isopropyl myristate, isopropyl palmitate, C₁₂-C₁₅ alkyl benzoate, hexyl laurate, diisopropyl adipate, isononyl isononanoate, 2-ethylhexyl palmitate, 2-octyldodecyl stearate, 2-octyldodecyl erucate, isostearyl isostearate or tridecyl trimellitate; octanoates, decanoates or ricinoleates of alcohols or polyalcohols, for instance propylene glycol dioctanoate; hydroxylated esters, for instance isostearyl lactate, octyl hydroxystearate, octyldodecyl hydroxystearate, diisostearyl malate, triisocetyl citrate, and fatty alcohol heptanoates, octanoates or decanoates; polyol esters, for instance propylene glycol dioctanoate, neopentyl glycol diheptanoate or diethylene glycol diisononanoate; and pentaerythritol esters, for instance pentaerythrityl tetraisostearate;
- fatty alcohols that are liquid at ambient temperature, containing a branched and/or unsaturated carbon chain containing from 12 to 26 carbon atoms, for instance octyldodecanol, isostearyl alcohol, 2-butyloctanol, 2-hexyldecanol, 2-undecylpentadecanol or oleyl alcohol;
- higher fatty acids such as oleic acid, linoleic acid or linolenic acid;
- carbonates;
- acetates;
- citrates;
- fluoro oils that are optionally partially hydrocarbon-based and/or silicone-based, for instance fluorosilicone oils, fluoro polyethers and fluorosilicones as described in the document EP-A-847 752;
- silicone oils, for instance non-volatile linear or cyclic polydimethylsiloxanes (PDMSs); polydimethylsiloxanes comprising alkyl, alkoxy or phenyl groups, which are pendant or at the end of a silicone chain, these groups containing from 2 to 24 carbon atoms; phenyl silicones, for instance phenyl trimethicones, phenyl dimethicones, phenyl trimethylsiloxy diphenyl siloxanes, diphenyl dimethicones, diphenyl methyldiphenyl trisiloxanes and 2-phenylethyl trimethylsiloxy silicates, and
- mixtures thereof.

### STRUCTURING AGENT

The compositions according to the invention comprising a fatty phase may also contain at least one agent for structuring said fatty phase, which may preferably be chosen from waxes, pasty compounds, thickeners and mineral or organic lipophilic gelling agents, and mixtures thereof.

It is understood that the amount of these compounds may be adjusted by a person skilled in the art so as not to harm the effect desired in the context of the present invention.

### Wax(es)

The wax is in general a lipophilic compound that is solid at ambient temperature (25°C), with a reversible solid/liquid change in state, having a melting point of greater than or equal to 30°C, which may be up to 200°C and in particular up to 120°C.

In particular, the waxes that are suitable for the invention may have a melting point of greater than or equal to 45°C and in particular of greater than or equal to 55°C.

Within the meaning of the invention, the melting point corresponds to the temperature of the most endothermic peak observed on thermal analysis (DSC) as described in Standard ISO 11357-3; 1999. The melting point of the wax may be measured using a differential scanning calorimeter (DSC), for example the calorimeter sold under the name MDSC 2920 by the company TA Instruments.

### The measuring protocol is as follows:

A sample of 5 mg of wax placed in a crucible is subjected to a first temperature rise ranging from -20°C to 100°C, at a heating rate of 10°C/minute, it is then cooled from 100°C to -20°C at a cooling rate of 10°C/minute and it is finally subjected to a second temperature rise ranging from -20°C to 100°C at a heating rate of 5°C/minute. During the second temperature rise, the variation in the difference in power absorbed by the empty crucible and by the crucible containing the sample of wax is measured as a function of the temperature. The melting point of the compound is the temperature value corresponding to the top of the peak of the curve representing the variation in the difference in power absorbed as a function of the temperature.

The waxes that may be used in the compositions according to the invention are chosen from waxes that are solid at ambient temperature of animal, vegetable, mineral or synthetic origin, and mixtures thereof.

As illustrations of waxes that are suitable for the invention, mention may be made especially of hydrocarbon waxes, for instance beeswax, lanolin wax, Chinese insect waxes, rice bran wax, carnauba wax, candelilla wax, ouricury wax, esparto wax, berry wax, shellac wax, Japan wax and sumac wax; montan wax, orange wax and lemon wax, refined sunflower wax sold under the name Sunflower Wax by Koster Keunen, microcrystalline waxes, paraffins and ozokerite; polyethylene waxes, the waxes obtained by the Fischer-Tropsch synthesis and waxy copolymers, and also esters thereof.

Mention may also be made of waxes obtained by catalytic hydrogenation of animal or vegetable oils containing linear or branched C₈-C₃₂ fatty chains. Mention may especially be made, among these waxes, of isomerized jojoba oil such as the trans-isomerized partially hydrogenated jojoba oil manufactured or sold by the company Desert Whale under the commercial reference Iso-Jojoba-50^{®}, hydrogenated sunflower oil, hydrogenated castor oil, hydrogenated coconut oil, hydrogenated lanolin oil and bis(1,1,1-trimethylolpropane) tetrastearate sold under the name Hest 2T-4S^{®} by the company Heterene.

Mention may also be made of silicone waxes (C₃₀₋₄₅ alkyl dimethicone) or fluoro waxes.

The waxes obtained by hydrogenation of castor oil esterified with cetyl alcohol, sold under the names Phytowax Castor 16L64^{®} and 22L73^{®} by the company Sophim, may also be used. Such waxes are described in Application FR-A-2 792 190.

A wax that may be used is a C₂₀-C₄₀ (alkyl (hydroxystearyloxy)stearate (the alkyl group containing from 20 to 40 carbon atoms), alone or as a mixture.

Such a wax is especially sold under the names "Kester Wax K 82 P^{®}", "Hydroxypolyester K 82 P^{®}" and "Kester Wax K 80 P^{®}" by the company Koster Keunen.

As microwaxes that may be used in the compositions according to the invention, mention may be made especially of carnauba microwaxes, such as the product sold under the name MicroCare 350^{®} by the company Micro Powders, microwaxes of synthetic wax, such as the product sold under the name MicroEase 114S^{®} by the company Micro Powders, microwaxes consisting of a mixture of carnauba wax and polyethylene wax, such as the products sold under the names Micro Care 300^{®} and 310^{®} by the company Micro Powders, microwaxes consisting of a mixture of carnauba wax and synthetic wax, such as the product sold under the name Micro Care 325^{®} by the company Micro Powders, polyethylene microwaxes, such as the products sold under the names Micropoly 200^{®}, 220^{®}, 220L^{®} and 250S^{®} by the company Micro Powders, the commercial products Performalene 400 Polyethylene and Performalene 500-L Polyethylene from New Phase Technologies, Performalene 655 Polyethylene or paraffin waxes, for instance the wax having the INCI name Microcrystalline Wax and Synthetic Wax and sold under the trade name Microlease by the company Sochibo; polytetrafluoroethylene microwaxes such as those sold under the names Microslip 519^{®} and 519 L^{®} by the company Micro Powders.

The composition according to the invention will preferably comprise a content of wax(es) ranging from 3% to 20% by weight relative to the total weight of the composition, in particular from 5% to 15% and more particularly from 6% to 15%. According to a particular form of the invention, in the context of anhydrous solid compositions in stick form, use will be made of polyethylene microwaxes in the form of crystallites with an aspect ratio at least equal to 2, and with a melting point ranging from 70 to 110°C and preferably from 70 to 100°C, in order to reduce or indeed even eliminate the presence of strata in the solid composition.

These crystallites in needle form and especially the dimensions thereof may be characterized visually according to the following method.

The wax is deposited on a microscope slide, which is placed on a hotplate. The slide and the wax are heated to a temperature generally at least 5°C higher than the melting point of the wax or of the mixture of waxes under consideration. At the end of melting, the liquid thus obtained and the microscope slide are allowed to cool in order to solidify. Observation of the crystallites is performed using a Leica DMLB100 optical microscope, with an objective lens selected as a function of the size of the objects to be viewed, and under polarized light. The dimensions of the crystallites are measured using image analysis software such as that sold by the company Microvision.

The crystallite polyethylene waxes in accordance with the invention preferably have an average length ranging from 5 to 10 mm. The term "average length" denotes the dimension given by the statistical particle size distribution at half the population, which is written as D50.

Use will be made more particularly of a mixture of Performalene 400 Polyethylene and Performalene 500-L Polyethylene waxes from New Phase Technologies.

### Pasty compounds

Within the meaning of the present invention, the term "pasty compound" is intended to denote a lipophilic fatty compound that undergoes a reversible solid/liquid change in state, which has in the solid form an anisotropic crystal organization, and that comprises, at a temperature of 23°C, a liquid fraction and a solid fraction.

The pasty compound is preferably chosen from synthetic compounds and compounds of vegetable origin. A pasty compound may be obtained by synthesis from starting materials of vegetable origin.

The pasty compound may be advantageously chosen from:
- lanolin and derivatives thereof,
- polymeric or non-polymeric silicone compounds,
- polymeric or non-polymeric fluoro compounds,
- vinyl polymers, especially:
- olefin homopolymers,
- olefin copolymers,
- hydrogenated diene homopolymers and copolymers,
- linear or branched oligomers, homopolymers or copolymers of alkyl (meth)acrylates preferably containing a C₈-C₃₀ alkyl group,
- oligomers, homopolymers and copolymers of vinyl esters containing C₈-C₃₀ alkyl groups, and
- oligomers, homopolymers and copolymers of vinyl ethers containing C₈-C₃₀ alkyl groups,
- fat-soluble polyethers resulting from the polyetherification between one or more C₂-C₁₀₀ and preferably C₂-C₅₀ diols,
- esters,
- mixtures thereof.

Among the esters, the following are especially preferred:
- esters of a glycerol oligomer, especially diglycerol esters, in particular condensates of adipic acid and of glycerol, for which some of the hydroxyl groups of the glycerols have reacted with a mixture of fatty acids such as stearic acid, capric acid, stearic acid and isostearic acid and 12-hydroxystearic acid, especially such as those sold under the brand name Softisan 649 by the company Sasol,
- the arachidyl propionate sold under the brand name Waxenol 801 by Alzo,
- phytosterol esters,
- fatty acid triglycerides and derivatives thereof,
- pentaerythritol esters,
- non-crosslinked polyesters resulting from the polycondensation between a linear or branched C4-C50 dicarboxylic acid or polycarboxylic acid and a C2-C50 diol or polyol,
- ester aliphatic esters resulting from the esterification of an aliphatic hydroxycarboxylic acid ester by an aliphatic carboxylic acid,
- polyesters resulting from the esterification, by a polycarboxylic acid, of an aliphatic hydroxycarboxylic acid ester, said ester comprising at least two hydroxyl groups, such as the products Risocast DA-H^{®} and Risocast DA-L^{®},
- esters of a diol dimer and of a diacid dimer, where appropriate esterified on their free alcohol or acid functional group(s) by acid or alcohol radicals, such as Plandool-G,
- mixtures thereof.

Among the pasty compounds of vegetable origin that will preferably be chosen is a mixture of oxyethylenated (5 OE) oxypropylenated (5 OP) pentaerythritol and soybean sterols, sold under the reference Lanolide by the company Vevy.

### Inorganic and lipophilic thickeners, gelling agents and suspending agents

Use may be made, as inorganic lipophilic thickener, gelling agent or suspending agent, of modified clays which are preferably chosen from hydrophobic modified montmorillonite clays, such as hydrophobic modified bentonites or hectorites. Examples that may be mentioned include the product Stearalkonium Bentonite (CTFA name) (reaction product of bentonite and the quaternary ammonium stearalkonium chloride) such as the commercial product sold under the name Tixogel MP 250 by the company Sud Chemie Rheologicals, United Catalysts Inc. or the product Disteardimonium Hectorite (CTFA name) (reaction product of hectorite and distearyldimonium chloride) sold under the name Bentone 38 or Bentone Gel by the company Elementis Specialities.
Mention may also be made of fumed silica optionally subjected to a hydrophobic surface treatment, the particle size of which is less than 1 mm. This is because it is possible to chemically modify the surface of the silica, by chemical reaction generating a reduced number of silanol groups present at the surface of the silica. It is especially possible to replace silanol groups with hydrophobic groups: a hydrophobic silica is then obtained. The hydrophobic groups may be trimethylsiloxyl groups, which are obtained especially by treating fumed silica in the presence of hexamethyldisilazane. Silicas thus treated are known as "silica silylate" according to the CTFA (8th Edition, 2000). They are sold, for example, under the references Aerosil R812^{®} by the company Degussa, CAB-O-SIL TS-530^{®} by the company Cabot, dimethylsilyloxyl or polydimethylsiloxane groups, which are obtained especially by treating fumed silica in the presence of polydimethylsiloxane or dimethyldichlorosilane. Silicas thus treated are known as "silica dimethyl silylate" according to the CTFA (8th Edition, 2000). They are sold, for example, under the references Aerosil R972^{®} and Aerosil R974^{®} by the company Degussa, and CAB-O-SIL TS-610^{®} and CAB-O-SIL TS-720^{®} by the company Cabot.

The hydrophobic fumed silica in particular has a particle size that may be nanometric to micrometric, for example ranging from about 5 to 200 nm.

### Organic and lipophilic thickeners, gelling agents and suspending agents

The organic lipophilic thickeners or gelling agents are, for example, partially or totally crosslinked elastomeric organopolysiloxanes of three-dimensional structure, for instance those sold under the names KSG6^{®}, KSG16^{®} and KSG18^{®} by the company Shin-Etsu, Trefil E-505C^{®} and Trefil E-506C^{®} by the company Dow Corning, Gransil SR-CYC^{®}, SR DMF10^{®}, SR-DC556^{®}, SR 5CYC gel^{®}, SR DMF 10 gel^{®} and SR DC 556 gel^{®} by the company Grant Industries and SF 1204^{®} and JK 113^{®} by the company General Electric; ethylcellulose, for instance the product sold under the name Ethocel^{®} by the company Dow Chemical; galactomannans comprising from one to six and in particular from two to four hydroxyl groups per saccharide, substituted by a saturated or unsaturated alkyl chain, for instance guar gum alkylated by C₁ to C₆ and in particular C₁ to C₃ alkyl chains, and mixtures thereof. Block copolymers of "diblock", "triblock" or "radial" type, of the polystyrene/polyisoprene or polystyrene/polybutadiene type, such as the products sold under the name Luvitol HSB^{®} by the company BASF, of the polystyrene/copoly(ethylene-propylene) type, such as the products sold under the name Kraton^{®} by the company Shell Chemical Co., or of the polystyrene/copoly(ethylene-butylene) type, and mixtures of triblock and radial (star) copolymers in isododecane, such as those sold by the company Penreco under the name Versagel^{®}, for instance the mixture of butylene/ethylene/styrene triblock copolymer and of ethylene/propylene/styrene star copolymer in isododecane (Versagel M 5960).

Lipophilic thickeners or gelling agents that may also be mentioned include polymers with a weight-average molecular weight of less than 100 000, comprising a) a polymer backbone with hydrocarbon repeat units containing at least one heteroatom, and optionally b) at least one optionally functionalized pendant fatty chain and/or at least one optionally functionalized terminal fatty chain, containing from 6 to 120 carbon atoms and being linked to these hydrocarbon units, as described in Applications WO-A-02/056847 and WO-A-02/47619, in particular polyamide resins (especially comprising alkyl groups containing from 12 to 22 carbon atoms) such as those described in US-A-5 783 657.

Among the lipophilic thickeners or gelling agents that may be used in the compositions according to the invention, mention may also be made of fatty acid esters of dextrin, such as dextrin palmitates, especially such as the products sold under the name Rheopearl TL^{®} or Rheopearl KL^{®} by the company Chiba Flour.

Silicone polyamides of the polyorganosiloxane type may also be used, such as those described in the documents US-A-5 874 069, US-A-5 919 441, US-A-6 051 216 and US-A-5 981 680.

These silicone polymers may belong to the following two families:
- polyorganosiloxanes comprising at least two groups capable of establishing hydrogen interactions, these two groups being located in the chain of the polymer, and/or
- polyorganosiloxanes comprising at least two groups capable of establishing hydrogen interactions, these two groups being located on grafts or branches.

The thickening agents, gelling agents and/or suspending agents are preferably present in amounts ranging from 0.1% to 15% by weight and more preferentially from 0.2% to 10% by weight relative to the total weight of the composition.

The amounts of these various constituents that may be present in the cosmetic composition according to the invention are those conventionally used in compositions for treating perspiration.

### MOISTURE ABSORBERS

It is also possible to add moisture absorbers, for instance perlites and preferably expanded perlites.

The perlites that may be used according to the invention are generally aluminosilicates of volcanic origin and have the composition:
70.0-75.0% by weight of silica SiO₂
12.0-15.0% by weight of aluminium oxide Al₂O₃
3.0-5.0% of sodium oxide Na₂O
3.0-5.0% of potassium oxide K₂O
0.5-2% of iron oxide Fe₂O₃ →
0.2-0.7% of magnesium oxide MgO
0.5-1.5% of calcium oxide CaO
0.05-0.15% of titanium oxide TiO₂

The perlite is ground, dried and then calibrated in a first step. The product obtained, known as perlite ore, is grey-coloured and has a size of about 100 mm.

The perlite ore is then expanded (1000°C/2 seconds) to give more or less white particles. When the temperature reaches 850-900°C, the water trapped in the structure of the material evaporates and brings about the expansion of the material relative to its original volume. The expanded perlite particles in accordance with the invention may be obtained via the expansion process described in Patent US 5 002 698.

Preferably, the perlite particles used will be ground; in this case, they are known as Expanded Milled Perlite (EMP). They preferably have a particle size defined by a median diameter D₅₀ ranging from 0.5 to 50 mm and preferably from 0.5 to 40 mm.

Preferably, the perlite particles used have a loose bulk density at 25°C ranging from 10 to 400 kg/m³ (Standard DIN 53468) and preferably from 10 to 300 kg/m³.

Preferably, the expanded perlite particles according to the invention have a water-absorbing capacity, measured at the wet point, ranging from 200% to 1500% and preferably from 250% to 800%.

The wet point corresponds to the amount of water that needs to be added to 1 g of particle in order to obtain a homogeneous paste. This method is derived directly from that of the oil uptake applied to solvents. The measurements are taken in the same manner by means of the wet point and the flow point, which have, respectively, the following definitions:
wet point: weight expressed in grams per 100 g of product corresponding to the production of a homogeneous paste during the addition of a solvent to a powder;
flow point: weight expressed in grams per 100 g of product at and above which the amount of solvent is greater than the capacity of the powder to retain it. This is reflected by the production of a more or less homogeneous mixture that flows over the glass plate.

The wet point and the flow point are measured according to the following protocol:

### Protocol for measuring the water absorption

### 1) Equipment used

Glass plate (25 x 25 mm)
Spatula (wooden shaft and metal part, 15 x 2.7 mm)
Silk-bristled brush
Balance

### 2) Procedure

The glass plate is placed on the balance and 1 g of perlite particles is weighed out. The beaker containing the solvent and the liquid sampling pipette is placed on the balance. The solvent is gradually added to the powder, the whole being regularly blended (every 3 to 4 drops) with the spatula.
The weight of solvent needed to obtain the wet point is noted. Further solvent is added and the weight which makes it possible to reach the flow point is noted. The average of three tests will be determined.

The expanded perlite particles sold under the trade names Optimat 1430 OR or Optimat 2550 by the company World Minerals will be used in particular.

### DEODORANT AGENTS

The compositions may in addition contain deodorant agents.

The term "deodorant active agent" refers to any substance that is capable of masking, absorbing, improving and/or reducing the unpleasant odour resulting from the decomposition of human sweat by bacteria.

The deodorant agents may be bacteriostatic agents or bactericides that act on underarm odour microorganisms, such as 2,4,4'-trichloro-2'-hydroxydiphenyl ether (^{®}Triclosan), 2,4-dichloro-2'-hydroxydiphenyl ether, 3',4',5'-trichlorosalicylanilide, 1-(3',4'-dichlorophenyl)-3-(4'-chlorophenyl)urea (^{®}Triclocarban) or 3,7,11-trimethyldodeca-2,5,10-trienol (^{®}Farnesol); quaternary ammonium salts such as cetyltrimethylammonium salts, cetylpyridinium salts, DPTA (1,3-diaminopropanetetraacetic acid), 1,2-decanediol (Symclariol from the company Symrise), glycerol derivatives, for instance caprylic/capric glycerides (Capmul MCM from Abitec), glyceryl caprylate or caprate (Dermosoft GMCY and Dermosoft GMC, respectively from Straetmans), polyglyceryl-2 caprate (Dermosoft DGMC from Straetmans), and biguanide derivatives, for instance polyhexamethylene biguanide salts. - chlorhexidine and salts thereof; 4-phenyl-4,4-dimethyl-2-butanol (Symdeo MPP from Symrise).

Among the deodorant active agents in accordance with the invention, mention may also be made of - zinc salts, for instance zinc salicylate, zinc gluconate, zinc pidolate; zinc sulfate, zinc chloride, zinc lactate, zinc phenolsulfonate; salicylic acid and derivatives thereof such as 5-(n-octanoyl)salicylic acid.

The deodorant active agents may be odour absorbers such as zinc ricinoleate, sodium bicarbonate; metallic or non-metallic zeolites, cyclodextrins or alum.

They may also be a chelating agent such as Dissolvine GL-47-S from Akzo Nobel, EDTA; DPTA.

They may also be a polyol such as glycerol or propane-1,3-diol (Zemea Propanediol sold by Dupont Tate and Lyle Bioproducts).

Alternatively, they may be an enzyme inhibitor such as triethyl citrate.
In the event of incompatibility or to stabilize them, some of the agents mentioned above may be incorporated into spherules, especially ionic or nonionic vesicles, and/or particles (capsules and/or spheres).

The deodorant agents may preferably be present in the compositions according to the invention in weight concentrations ranging from 0.01% to 15% by weight relative to the total weight of the composition A or B.

### ORGANIC POWDER

According to one particular form of the invention, the compositions A and/or B according to the invention will also contain an organic powder.

In the present application, the term "organic powder" means any solid that is insoluble in the medium at ambient temperature (25°C).

As organic powders that may be used in the composition of the invention, examples that may be mentioned include polyamide particles and especially those sold under the Orgasol names by the company Atochem; nylon-6,6 fibres, especially the polyamide fibres sold by Etablissements P Bonte under the name Polyamide 0.9 Dtex 0.3 mm (INCI name: Nylon-6,6 or Polyamide 6,6) with a mean diameter of 6 mm, a weight of about 0.9 dtex and a length ranging from 0.3 mm to 1.5 mm; polyethylene powders; microspheres based on acrylic copolymers, such as those made of ethylene glycol dimethacrylate/lauryl methacrylate copolymer, sold by the company Dow Corning under the name Polytrap; polymethyl methacrylate microspheres, sold under the name Microsphere M-100 by the company Matsumoto or under the name Covabead LH85 by the company Wackherr; hollow polymethyl methacrylate microspheres (particle size: 6.5-10.5 mm) sold under the name Ganzpearl GMP 0800 by Ganz Chemical; methyl methacrylate/ethylene glycol dimethacrylate copolymer microbeads (size: 6.5-10.5 m) sold under the name Ganzpearl GMP 0820 by Ganz Chemical or Microsponge 5640 by the company Amcol Health & Beauty Solutions; ethylene-acrylate copolymer powders, such as those sold under the name Flobeads by the company Sumitomo Seika Chemicals; expanded powders such as hollow microspheres and especially microspheres formed from a terpolymer of vinylidene chloride, acrylonitrile and methacrylate and sold under the name Expancel by the company Kemanord Plast under the references 551 DE 12 (particle size of about 12 mm and density of 40 kg/m3), 551 DE 20 (particle size of about 30 mm and density of 65 kg/m3), 551 DE 50 (particle size of about 40 mm), or the microspheres sold under the name Micropearl F 80 ED by the company Matsumoto; powders of natural organic materials such as starch powders, especially of crosslinked or non-crosslinked maize, wheat or rice starch, such as the powders of starch crosslinked with octenylsuccinic anhydride, sold under the name Dry-Flo by the company National Starch; silicone resin microbeads such as those sold under the name Tospearl by the company Toshiba Silicone, especially Tospearl 240; amino acid powders such as the lauroyllysine powder sold under the name Amihope LL-11 by the company Ajinomoto; particles of wax microdispersion, which preferably have mean sizes of less than 1 mm and especially ranging from 0.02 mm to 1 mm, and which are formed essentially from a wax or a mixture of waxes, such as the products sold under the name Aquacer by the company Byk Cera, and especially: Aquacer 520 (mixture of synthetic and natural waxes), Aquacer 514 or 513 (polyethylene wax), Aquacer 511 (polymeric wax), or such as the products sold under the name Jonwax 120 by the company Johnson Polymer (mixture of polyethylene wax and paraffin wax) and under the name Ceraflour 961 by the company Byk Cera (micronized modified polyethylene wax); and mixtures thereof.

### ADDITIVES

The compositions according to the invention may also comprise cosmetic adjuvants chosen from softeners, antioxidants, opacifiers, stabilizers, moisturizers, vitamins, bactericides, preserving agents, polymers, fragrances, propellants or any other ingredient usually used in cosmetics for this type of application.

Needless to say, a person skilled in the art will take care to select this or these optional additional compounds such that the advantageous properties intrinsically associated with the cosmetic composition in accordance with the invention are not, or are not substantially, adversely affected by the envisaged addition(s).

### AEROSOLS

The compositions according to the invention may also be pressurized and may be packaged in an aerosol device formed by:
(A) a container comprising an antiperspirant composition as defined previously,
(B) at least one propellant and a means for dispensing said aerosol composition.

The propellants generally used in products of this type and that are well known to those skilled in the art are, for instance, dimethyl ether (DME); volatile hydrocarbons such as n-butane, propane, isobutane and mixtures thereof, optionally with at least one chlorohydrocarbon and/or fluorohydrocarbon; among the latter, mention may be made of the compounds sold by the company DuPont de Nemours under the names Fréon^{®} and Dymel^{®}, and in particular monofluorotrichloromethane, difluorodichloromethane, tetrafluorodichloroethane and 1,1-difluoroethane sold especially under the trade name Dymel 152 A by the company DuPont. Carbon dioxide, nitrous oxide, nitrogen or compressed air may also be used as propellant.

The compositions containing perlite particles as defined previously and the propellant(s) may be in the same compartment or in different compartments in the aerosol container. According to the invention, the concentration of propellant generally varies from 5% to 95% by weight of pressurized composition, and more preferentially from 50% to 85% by weight relative to the total weight of the pressurized composition.

The dispensing means, which forms a part of the aerosol device, is generally formed by a dispensing valve controlled by a dispensing head, which itself comprises a nozzle via which the aerosol composition is vaporized. The container containing the pressurized composition may be opaque or transparent. It may be made of glass, polymer or metal, optionally coated with a protective varnish coat.

The examples that follow serve to illustrate the present invention.

### Example 1

| | |
|---|---|
| Calcium chloride | 7.35% |
| Water q.s. for | 100 |

The antiperspirant effectiveness of the composition according to Example 1 was evaluated on a panel of 22 women, according to the following protocol:
i) Two times 8 areas (4 × 5 cm²) are delimited on either side of the spinal column. Each product area has a corresponding symmetrical untreated control area.
ii) The composition is applied with occlusion for 1 hour, for 4 consecutive days, with gentle massaging of the antiperspirant products. Total amount applied: 3.75 mg/cm².
   24 hours after the last application:
iii) The back is washed with water to remove any remaining trace of product; cellulose squares are fixed onto the various areas, and the person is then made to sweat in a sauna for 15 minutes at 80°C.
iv) The amount of sweat is evaluated by weighing the cellulose squares before and after sweating.

Under these test conditions, a significant reduction in sweating of 25% to 35% is measured.

### Example 2

| | |
|---|---|
| Calcium chloride | 11.65% |
| Water q.s. for | 100 |

The antiperspirant effectiveness of the composition according to Example 2 was evaluated on a panel of 22 women, according to the following protocol:
i) Two times 8 areas (4 × 5 cm²) are delimited on either side of the spinal column. Each product area has a corresponding symmetrical untreated control area.
ii) The composition is applied with occlusion for 1 hour, for 4 consecutive days, with gentle massaging of the antiperspirant products. Total amount applied: 3.75 mg/cm².
   24 hours after the last application:
iii) The back is washed with water to remove any remaining trace of product; cellulose squares are fixed onto the various areas, and the person is then made to sweat in a sauna for 15 minutes at 80°C.
iv) The amount of sweat is evaluated by weighing the cellulose squares before and after sweating.

Under these test conditions, a significant reduction in sweating of 35% to 45% is measured.

### Example 3

| | |
|---|---|
| Calcium chloride | 3.68% |
| Water q.s. for | 100 |

The antiperspirant effectiveness of the composition according to Example 3 was evaluated on a panel of 22 women, according to the following protocol:
i) Two times 8 areas (4 × 5 cm²) are delimited on either side of the spinal column. Each product area has a corresponding symmetrical untreated control area.
ii) The composition 3 is applied with occlusion for 1 hour, for 4 consecutive days, with gentle massaging of the antiperspirant products. Total amount applied: 3.75 mg/cm².
   24 hours after the last application:
iii) The back is washed with water to remove any remaining trace of product; cellulose squares are fixed onto the various areas, and the person is then made to sweat in a sauna for 15 minutes at 80°C.
iv) The amount of sweat is evaluated by weighing the cellulose squares before and after sweating.

Under these test conditions, a significant reduction in sweating is measured.

### Example 4: O/W emulsion

| Phase | **Ingredients** | **Amounts in % by weight** |
|---|---|---|
| A | DIMETHICONE | 0.5 |
| | CETEARYL ALCOHOL | 2.5 |
| | CETEARETH-33 | 1.25 |
| | PPG-15 STEARYL ETHER | 3 |
| B | CALCIUM CHLORIDE | 11.65 |
| | AQUA | 81.025 |
| | IODOPROPYNYL BUTYLCARBAMATE | 0.075 |

The phases A and B are heated at 75°C separately. Phase 1 is poured into phase 2 and then emulsified with a rotor-stator for 15 min. The heating is stopped and the formula is stirred with a deflocculating agitator. The preserving agent is added and the formula is left to cool, while being stirred in the deflocculating agitator, to ambient temperature.

### Example 5: Aqueous-alcoholic gel

| **Ingredients** | **Amounts in % by weight** |
|---|---|
| CALCIUM CHLORIDE | 7.35 |
| HYDROXYETHYL CELLULOSE | 0.6 |
| DENAT. ALCOHOL | 20 |
| AQUA | 72.05 |

The ingredients are added one by one, being stirred until homogenization is achieved.

### Example 6: Anhydrous aerosol fluid

| **Phase** | **Ingredients** | **Amounts in % by weight** |
|---|---|---|
| A | ISOPROPYL PALMITATE | 6 |
| | DIMETHICONE | 61.75 |
| | DIMETHICONE (and) DIMETHICONOL | 11 |
| | TRIETHYL CITRATE | 7 |
| B | STEARALKONIUM BENTONITE | 2.6 |
| C | CALCIUM CHLORIDE | 11.65 |

Using a rotor-stator, the ingredients of phase A are mixed. Compound C is added and the stirring is stopped for five minutes. The formula is then stirred again using a rotor-stator for 10 minutes and then compound C is added. The stirring is maintained until homogenization is achieved.

### Example 7: Stick

### emulsion[0164]

| **Phase** | **Ingredients** | **Amounts in % by weight** |
|---|---|---|
| A | CALCIUM CHLORIDE | 11.67 |
| | AQUA | 55.328 |
| | CI 42090 | 0.002 |
| B | BEHENETH-10 | 2 |
| C | SYNTHETIC WAX | 10 |
| D | PHENOXYETHANOL | 0.5 |
| | ISOPROPYL PALMITATE | 9 |
| | DIMETHICONE | 5.7 |
| | CETYL PEG/PPG-10/1 DIMETHICONE | 2 |
| | PEG/PPG-18/18 DIMETHICONE | 2 |
| | PENTYLENE GLYCOL | 0.5 |
| | POLYGLYCERYL-3 DIISOSTEARATE | 0.3 |

| **Phase** | **Ingredients** | **Amounts in % by weight** |
|---|---|---|
| E | PERLITE | 0.2 |
| | NYLON-12 | 0.5 |
| | ACRYLATES COPOLYMER | 0.3 |

The ingredients of phase A are mixed and heated. Compound B is added at 55°C, then compound C is added at 85°C to result in an intermediate formula. Phase D is melted separately, then it is added to the intermediate formula. The new mixture thus obtained is heated at 90°C and phase E is introduced. The whole assembly is homogenized before casting at 90°C, then cooled for 45 minutes at 4°C.

## Claims

1. Non-therapeutic use of a cosmetic composition comprising, in a cosmetically acceptable medium, calcium chloride as antiperspirant agent, for treating human perspiration, in order to reduce the flow of sweat, said composition containing no aluminium and/or zirconium antiperspirant halogenated salt, nor compound capable of reacting with said calcium chloride in order to produce together on the skin, in situ, an antiperspirant effect.

2. Use of a cosmetic composition according to claim 1, wherein the concentration of calcium chloride varies from 0.1% to 20% by weight and more preferentially from 0.5% to 15% by weight relative to the total weight of the composition.

## Patentansprüche

1. Nichttherapeutische Verwendung einer kosmetischen Zusammensetzung, die in einem kosmetisch annehmbaren Medium Calciumchlorid als schweißhemmendes Mittel umfasst, zur Behandlung von menschlicher Schweißbildung, um den Schweißfluss zu verringern, wobei die Zusammensetzung kein schweißhemmendes halogeniertes Aluminium- und/oder Zirkoniumsalz und keine Verbindung enthält, die fähig ist, mit dem Calciumchlorid zu reagieren, um zusammen auf der Haut *in situ* eine schweißhemmende Wirkung zu erzeugen.

2. Verwendung einer kosmetischen Zusammensetzung gemäß Anspruch 1, wobei die Konzentration von Calciumchlorid von 0,1 Gew.-% bis 20 Gew.-% und bevorzugter von 0,5 Gew.-% bis 15 Gew.-%, bezogen auf das Gesamtgewicht der Zusammensetzung, variiert.

## Revendications

1. Utilisation non thérapeutique d'une composition cosmétique comprenant, dans un milieu acceptable sur le plan cosmétique, du chlorure de calcium en tant qu'agent anti-transpirant, pour traiter la transpiration humaine, afin de réduire l'écoulement de sueur, ladite composition ne contenant pas de sel halogéné anti-transpirant d'aluminium et/ou de zirconium, ni de composé capable de réagir avec ledit chlorure de calcium de façon à produire conjointement sur la peau, *in situ,* un effet anti-transpirant.

2. Utilisation d'une composition cosmétique selon la revendication 1, dans laquelle la concentration de chlorure de calcium varie de 0,1 % à 20 % en poids et plus préférentiellement de 0,5 % à 15 % en poids par rapport au poids total de la composition.
